# EUROPEAN PATENT APPLICATION

(11) **EP 4 545 105 A1**
(43) Date of publication of application: **30.04.2025**
(21) Application number: 24205731.3
(22) Date of filing: 10.10.2024
(51) Int. Cl.: A61L 27/12, A61L 27/46, A61L 27/54

(54) **BONE REGENERATION MATERIAL, METHOD FOR MANUFACTURING BONE REGENERATION MATERIAL, AND BONE REGENERATION MATERIAL MANUFACTURING DEVICE**

(30) Priority: 24.10.2023 JP 2023182880
(71) Applicant: Tohoku University, Sendai-shi, Miyagi 980-8577 (JP); Japan Fine Ceramics Co., Ltd., Sendai, Miyagi Pref. 981-3206 (JP)
(72) Inventor: SUZUKI, Osamu, Sendai-shi, Miyagi, 980-8577 (JP); HAMAI, Ryo, Sendai-shi, Miyagi, 980-8577 (JP); TSUCHIYA, Kaori, Sendai-shi, Miyagi, 980-8577 (JP); MORI, Yu, Sendai-shi, Miyagi, 980-8577 (JP); KANABUCHI, Ryuichi, Sendai-shi, Miyagi, 980-577 (JP); KURIYAMA, Yasuaki, Sendai-shi, Miyagi, 980-8577 (JP); TSUBOI, Yuki, Sendai-shi, Miyagi, 980-8577 (JP); CHAIARIYAKUL, Danupong, Sendai-shi, Miyagi, 980-8577 (JP); HOSOYA, Keizo, Sendai, Miyagi Pref., 981-3206 (JP); TAHARA, Naoki, Yokohama-shi, Kanagawa, 2206001 (JP)
(74) Representative: Becker Kurig & Partner Patentanwälte mbB

(57) **Abstract**

To provide a bone regeneration material, a method for manufacturing a bone regeneration material, and a bone regeneration material manufacturing device (1) that control the adsorption amount of protein with respect to octacalcium phosphate. A bone regeneration material is formed by a complex. The complex includes: a base material including octacalcium phosphate; and functional molecules, adsorbed to the octacalcium phosphate.

## Description

### BACKGROUND

### Technical Field

The disclosure relates to a bone regeneration material, a method for manufacturing a bone regeneration material, and a bone regeneration material manufacturing device.

### Description of Related Art

Conventionally, a method is known for increasing the adsorption amount of protein with respect to octacalcium phosphate by facilitating the precipitation of new octacalcium phosphate on the seed crystals of octacalcium phosphate under the supersaturated condition (see, for example, Patent Document 1).

### [Prior Art Document(s)]

### [Patent Document(s)]

[Patent Document 1] Japanese Laid-open No. 2021-16724

### SUMMARY

### [Issues to be solved by the invention]

However, the method described in Patent Document 1 exhibits an issue that it is not possible to control the adsorption amount of protein with respect to octacalcium phosphate, and it is not possible to adjust the adsorption amount of protein according to the intended use.

The disclosure is made in view of the above circumstances and aims to provide a bone regeneration material, a method for manufacturing a bone regeneration material, and a bone regeneration material manufacturing device that control the adsorption amount of protein with respect to octacalcium phosphate.

### [Means for solving the issues]

The disclosure has the following aspects.
[1] A bone regeneration material is formed by a complex. The complex includes: a base material including octacalcium phosphate; and functional molecules, adsorbed to the octacalcium phosphate.
[2] In the bone regeneration material according to [1], the functional molecules are selected from at least one of growth factors, serum-derived protein.
[3] In the bone regeneration material according to [2], the base material includes a polylactic-co-glycolic acid copolymer.
[4] A method for manufacturing a bone regeneration material, includes: preparing a phosphate-buffered saline solution by dissolving functional molecules in a phosphate-buffered saline; bringing the phosphate-buffered saline solution into contact with a base material including octacalcium phosphate to adsorb the functional molecules to the octacalcium phosphate; and freeze-drying the base material including the octacalcium phosphate with the adsorbed functional molecules.
[5] A method for manufacturing a bone regeneration material, includes: dissolving functional molecules in a buffer solution that is saturated or supersaturated with respect to octacalcium phosphate to prepare a functional molecule-containing buffer solution; immersing a base material including octacalcium phosphate in the functional molecule-containing buffer solution to adsorb the functional molecules to the octacalcium phosphate; and freeze-drying the base material including the octacalcium phosphate with the adsorbed functional molecules.
[6] A bone regeneration material manufacturing device includes: a reaction vessel, accommodating a slurry including a base material that includes octacalcium phosphate; an acid/base supply unit, supplying acid or base to the slurry in the reaction vessel; a calcium ion supply unit, supplying calcium ions to the slurry in the reaction vessel; an inorganic phosphate ion supply unit, supplying inorganic phosphate ions to the slurry in the reaction vessel; a functional molecule supply unit, supplying functional molecules to the slurry in the reaction vessel; a functional molecule concentration unit, after the functional molecules are adsorbed onto the base material in the reaction vessel, recovering and concentrating remaining functional molecules in a supernatant of the slurry among the functional molecules, and supplying the recovered and concentrated functional molecules to the reaction vessel again; a pH electrode, measuring a pH of the slurry in the reaction vessel; a calcium ion electrode, measuring a concentration of calcium ions of the slurry in the reaction vessel; and a control unit, calculating a degree of supersaturation with respect to the octacalcium phosphate for the slurry based on the pH of the slurry in the reaction vessel measured by the pH electrode and the concentration of the calcium ions of the slurry in the reaction vessel measured by the calcium ion electrode, and, based on the degree of supersaturation, transmitting an instruction to the acid/base supply unit, the calcium ion supply unit, and the inorganic phosphate ion supply unit to supply the acid or base, the calcium ions, and the inorganic phosphate ions to the slurry in the reaction vessel, so that the degree of supersaturation of the slurry in the reaction vessel becomes a set value.

### [Inventive effects]

According to the disclosure, a bone regeneration material, a method for manufacturing a bone regeneration material, and a bone regeneration material manufacturing device that control the adsorption amount of protein with respect to octacalcium phosphate can be provided.

### BRIEF DESCRIPTION OF THE DRAWINGS

FIG. 1 is a schematic diagram showing a bone regeneration material manufacturing device according to an embodiment of the disclosure.
FIG. 2 is a diagram describing a mechanism of facilitating adsorption of functional molecules onto a base material including octacalcium phosphate (OCP) in a buffer solution supersaturated with respect to OCP.
FIG. 3 is a diagram describing a mechanism of facilitating adsorption of functional molecules onto a base material including OCP in a buffer solution supersaturated with respect to OCP.
FIG. 4 is a diagram showing a ratio of a newly formed bone area with respect to a defect area in Example 1.
FIG. 5 is a diagram showing a ratio of a remaining OCP granule area with respect to the defect area in Example 1.
FIG. 6 is a diagram showing the DNA concentrations of cells at the 14^{th} day or the 21^{st} day after the start of culture in Example 2.
FIG. 7 is a diagram showing the ALP activity at the 14^{th} day or the 21^{st} day after the start of culture in Example 2.
FIG. 8 is a diagram showing a relationship between the initial concentration of cytochrome c in a buffer solution saturated with respect to OCP or a buffer solution supersaturated with respect to OCP, and the adsorption amount of cytochrome c onto OCP in Example 4.
FIG. 9 is a diagram showing the results of the crystal structure of OCP with adsorbed cytochrome c through an X-ray diffraction analysis in Example 4.
FIG. 10 is a transmission electron microscope image of OCP crystals before adsorption of cytochrome c in Example 5.
FIG. 11 is a transmission electron microscope image of OCP crystals with cytochrome c adsorbed by using a cytochrome c-containing buffer solution E in Example 5.
FIG. 12 is a transmission electron microscope image of OCP crystals with cytochrome c adsorbed by using a cytochrome c-containing buffer solution F in Example 5.
FIG. 13 is a diagram showing adsorption isotherms of fetuin onto OCP in fetuin-containing 0.5Ca0.5Pi buffer solutions and fetuin-containing 3.0Ca1.0Pi buffer solutions in Example 6.
FIG. 14 is a diagram showing measurement results of CD spectra of fetuin in the 0.5Ca0.5Pi buffer solutions and the 3.0Ca1.0Pi buffer solutions before and after immersion of OCP in Example 6.
FIG. 15 is a diagram showing secondary structure elements of fetuin in the 0.5Ca0.5Pi buffer solutions and the 3.0Ca1.0Pi buffer solutions in Example 6.

### DESCRIPTION OF THE EMBODIMENTS

The disclosure describes embodiments of a bone regeneration material, a method for manufacturing a bone regeneration material, and a bone regeneration material manufacturing device.

It should be noted that the embodiments are specifically explained to provide a better understanding of the spirit of the disclosure and, unless otherwise specified, do not limit the disclosure.

### [Bone regeneration material]

The bone regeneration material according to an embodiment of the disclosure is formed by a complex including a base material including octacalcium phosphate and functional molecules (e.g. biofunctional molecules) adsorbed to the octacalcium phosphate.

The shape of the complex can be arbitrarily set in consideration of the shape and the size, etc., of the affected area to which the complex is filled. For example, the complex may be in a rectangular parallelepiped (block) shape, a cubic shape, a cylindrical shape, a tablet shape, or a granular shape.

### (Base material)

The base material includes octacalcium phosphate (Ca₈H₂(PO₄)₆ • 5H₂O, hereinafter also referred to as "OCP"). The base material may be composed solely of OCP, or may include OCP and other components.

In the case where the base material includes OCP and other components, a polylactic-co-glycolic acid (hereinafter also referred to as "PLGA") copolymer may serve as an other component.

In the case where the base material includes OCP and PLGA, the ratio between OCP and PLGA is not particularly limited. However, for example, based on the total amount of OCP and PLGA, the content of OCP is preferably 20 mass% or more and 65 mass% or less, and more preferably 20 mass% or more and 40 mass% or less. Additionally, it is preferable that the content of PLGA is 35 mass% or more and 80 mass% or less, and more preferably 60 mass% or more and 80 mass% or less. This allows for the balance between the osteoconductivity of OCP and the bioabsorbability of PLGA, resulting in a bone regeneration material with excellent osteoconductivity.

OCP is a conventional substance and can be prepared, for example, by LeGeros' dropwise method (LeGeros RZ, Calcif Tissue Int 37:194-197, 1985) or by a method using a synthesis device (triple-tube apparatus) described in Japanese Patent No. 3115642.

### (Functional molecule)

The functional molecules are adsorbed to the base material and, in addition to enhancing bone regeneration by OCP, facilitates cell migration and proliferation, etc. For example, as reported in Tohoku J Exp Med 164:37-50, 1991 and Bone Miner 20:151-166, 1993, it is considered that the functional molecules accumulate and exert functions in spaces between OCP crystals.

As the functional molecules, growth factors, serum-derived proteins may be adopted. As the growth factors, examples may include stromal cell-derived factor 1 (SDF-1), bone morphogenetic protein (BMP-2), vascular endothelial growth factor (VEGF), and others. For example, by using SDF-1 adsorbed to the base material, an effect of facilitating the migration of mesenchymal stem cells (MSCs) that can differentiate into osteoblasts can be expected.

As serum-derived proteins, examples may include fetuin (α2-HS glycoproteins), albumin, fibronectin, etc. For example, by using fetuin adsorbed to the base material, an effect of facilitating bone formation by binding calcium ions in the surrounding and phosphate ions can be obtained. By using fibronectin adsorbed to the base material, an effect of enhancing cell adhesion can be obtained.

The functional molecules can be used alone or in combination of two or more types. By using a combination of two or more types of functional molecules, two or more effects can be brought to a living body.

The adsorption amount of the functional molecules to the base material is preferably 50 ng or more and 70 µg or less, more preferably 100 ng or more and 60 µg or less, and even more preferably 200 ng or more and 20 µg or less per unit area (cm²) of the base material. In the case where the adsorption amount of the functional molecules is equal to or greater than the lower limit, bone regeneration is expected to be facilitated. In the case where the adsorption amount of the functional molecules is less than the lower limit, the bone regeneration facilitating effect cannot be obtained. In the case where the adsorption amount of the functional molecules exceeds the upper limit, cytotoxicity or inflammation may be induced, and a sufficient bone regeneration effect cannot be obtained.

The bone regeneration material of the embodiment may include components generally included in bone regeneration materials to the extent that such components do not inhibit the effects of the disclosure. Such components may include, for example, collagen, gelatin, alginic acid, hyaluronic acid, chitosan, biodegradable polymers (polylactic acid, polylactic acid-polyethylene glycol copolymer, etc.), biodegradable calcium phosphates (β-tricalcium phosphate (β-TCP), α-tricalcium phosphate (α-TCP), tetracalcium phosphate (Ca₄(PO₄)₂O; TTCP), calcium hydrogen phosphate (CaHPO₄; DCP), calcium hydrogen phosphate dihydrate (CaHPO₄ • 2H₂O; DCPD), low crystalline hydroxyapatite (Ca₁₀(PO₄)₆(OH)₂; HA), nano HA, carbonate-containing HA, etc.), and non-biodegradable materials (HA ceramics, etc.).

According to the bone regeneration material of the embodiment, since the bone regeneration material is formed by the complex including the base material containing OCP and the functional molecules adsorbed to OCP, in the case of application to a living body, in addition to enhancing bone regeneration by OCP, the effect of the functional molecules can also be brought forth. Furthermore, the bone regeneration material of the embodiment can be a material in which the adsorption amount of the functional molecules is controlled through the manufacturing method of the bone regeneration material to be described afterwards.

### [Manufacturing method of the bone regeneration material]

### (First embodiment)

The manufacturing method of the bone regeneration material according to an embodiment of the disclosure includes: a step of dissolving the functional molecules in a phosphate-buffered saline to prepare a phosphate-buffered saline solution (hereinafter referred to as "first step"); a step of bringing the phosphate-buffered saline solution into contact with the base material including octacalcium phosphate to adsorb the functional molecules to the octacalcium phosphate (hereinafter referred to as "second step"); and a step of freeze-drying the base material including the octacalcium phosphate to which the functional molecules are adsorbed (hereinafter referred to as "third step") .

### (First step)

The concentration of the phosphate-buffered saline is preferably 1.0 mmol/L or more and 30.0 mmol/L or less, and more preferably 5.0 mmol/L or more and 15.0 mmol/L or less. When the concentration of the phosphate-buffered saline is less than the lower limit, the pH buffering capacity decreases. When the concentration of the phosphate-buffered saline exceeds the upper limit, there is a possibility of cytotoxicity due to precipitated phosphate.

The content (concentration) of the functional molecules in the phosphate-buffered saline solution is preferably 3.0 µmol/L or more and 30.0 µmol/L or less, more preferably 4.0 µmol/L or more and 10.0 µmol/L or less, and even more preferably 4.5 µmol/L or more and 7.5 µmol/L or less. In the case where the content of the functional molecules is less than the lower limit, the loading amount of the functional molecules is small, and a sufficient bone regeneration effect may not be obtained. In the case where the content of the functional molecules exceeds the upper limit, cytotoxicity or inflammation may be induced, and a sufficient bone regeneration effect cannot be obtained.

### (Second step)

In the second step, bringing the phosphate-buffered saline solution into contact with the base material including OCP includes, for example, dropping the phosphate-buffered saline solution onto the base material including OCP, or immersing the base material including OCP in the phosphate-buffered saline solution.

In the case of immersing the base material including OCP in the phosphate-buffered saline solution, the immersion time is not particularly limited, but may be 1 second or more and 10 minutes or less, for example.

In the case of dropping the phosphate-buffered saline solution onto the base material including OCP, the amount of the phosphate-buffered saline solution to be dropped per unit mass (mg) of the base material is preferably 2.0 µL or more and 8.0 µL or less, more preferably 3.0 µL or more and 7.0 µL or less, and even more preferably 4.0 µL or more and 6.0 µL or less. When the dropping amount of the phosphate-buffered saline solution is equal to or more than the lower limit, the functional molecules can be uniformly loaded on the surface of OCP. When the dropping amount of the phosphate-buffered saline solution is equal to or less than the upper limit, the dissolution of OCP crystals can be suppressed.

In the case of immersing the base material including OCP in the phosphate-buffered saline solution, the mass (mg) of the base material including OCP to be added per unit volume (µL) of the phosphate-buffered saline solution is preferably 0.10 mg or more and 0.50 mg or less, more preferably 0.15 mg or more and 0.30 mg or less, and even more preferably 0.15 mg or more and 0.25 mg or less, in terms of OCP. When the mass of the base material including OCP is equal to or more than the lower limit, the dissolution of OCP crystals can be suppressed. When the mass of the base material including OCP is equal to or less than the upper limit, the functional molecules can be uniformly loaded on OCP.

### (Third step)

In the third step, the base material including OCP with the adsorbed functional molecules may be pre-freezed before the base material including OCP with the adsorbed functional molecules is freeze-dried. The temperature for pre-freezing is may be -196°C or higher and -10°C or lower.

The freeze-drying time is not particularly limited, and may be 12 hours or more and 120 hours or less, for example.

According to the bone regeneration material of the embodiment, the adsorption amount of the functional molecule to OCP can be controlled because the functional molecules are adsorbed to OCP by using the phosphate-buffered saline solution in which the content of the functional molecule is adjusted.

### (Second embodiment)

The method for manufacturing the bone regeneration material according to an embodiment of the disclosure includes a step of dissolving the functional molecules in a buffer solution that is saturated or supersaturated with respect to octacalcium phosphate to prepare a functional molecule-containing buffer solution (hereinafter referred to as the "first step"); a step of immersing the base material including octacalcium phosphate in the functional molecule-containing buffer solution to adsorb the functional molecule to the octacalcium phosphate (hereinafter referred to as "second step"); a step of freeze-drying the base material including the octacalcium phosphate to which the functional molecules are adsorbed (hereinafter referred to as "third step") .

### (First step)

As an example of the buffer solution saturated with respect to OCP, examples include a solution including calcium ions (Ca²⁺), inorganic phosphate ions, and a tris(hydroxymethyl)aminomethane -HCl (tris-HCl) buffer, with a pH of 5.0 or higher and 8.0 or lower.

In the buffer solution saturated with respect to OCP, the concentration of the calcium ions is preferably 0.1 mmol/L or more and 1.0 mmol/L or less, and more preferably 0.4 mmol/L or more and 0.6 mmol/L or less. When the concentration of the calcium ions is less than the lower limit, the OCP, which is the base material, dissolves. When the concentration of the calcium ions exceeds the upper limit, HA precipitates.

In the buffer solution saturated with respect to OCP, the concentration of inorganic phosphate ions is preferably 0.1 mmol/L or more and 1.0 mmol/L or less, and more preferably 0.4 mmol/L or more and 0.6 mmol/L or less. When the concentration of inorganic phosphate ions is less than the lower limit, the OCP, which is the base material, dissolves. When the concentration of inorganic phosphate ions exceeds the upper limit, HA precipitates.

In the buffer solution saturated with respect to OCP, the concentration of tris(hydroxymethyl)aminomethane in the tris-HCl buffer is preferably 50 mmol/L or more and 300 mmol/L or less, and more preferably 100 mmol/L or more and 200 mmol/L or less. When the concentration of the tris-HCl buffer is less than the lower limit, the pH buffering capacity is lost. If the concentration of the tris-HCl buffer exceeds the upper limit, there is a possibility of cytotoxicity due to the residual of tris(hydroxymethyl)aminomethane.

The pH of the buffer solution saturated with respect to OCP is preferably 5.0 or higher and 8.0 or lower, more preferably 6.8 or higher and 8.0 or lower, and even more preferably 7.2 or higher and 7.6 or lower. When the pH is less than the lower limit, OCP dissolves. When the pH exceeds the upper limit, HA precipitates.

As an example of the buffer solution supersaturated with respect to OCP, examples include a solution including calcium ions (Ca²⁺), inorganic phosphate ions, and a tris-HCl buffer, with a pH of 5.0 or higher and 8.0 or lower.

In the buffer solution supersaturated with respect to OCP, the concentration of the calcium ions is preferably 0.5 mmol/L or more and 5.0 mmol/L or less, more preferably 1.0 mmol/L or more and 4.0 mmol/L or less, and even more preferably 2.5 mmol/L or more and 3.5 mmol/L or less. When the concentration of the calcium ions is within the range, new OCP crystals precipitate on the OCP crystals. When the concentration of the calcium ions is less than the lower limit, the OCP crystals dissolve. When the concentration of the calcium ions exceeds the upper limit, HA precipitates.

In the buffer solution supersaturated with respect to OCP, the concentration of inorganic phosphate ions is preferably 0.5 mmol/L or more and 5.0 mmol/L or less, more preferably 0.75 mmol/L or more and 4.0 mmol/L or less, and even more preferably 1.0 mmol/L or more and 3.0 mmol/L or less. When the concentration of inorganic phosphate ions is within the range, new OCP crystals precipitate on the OCP crystals. When the concentration of inorganic phosphate ions is less than the lower limit, the OCP crystals dissolve. When the concentration of inorganic phosphate ions exceeds the upper limit, HA precipitates.

In the buffer solution supersaturated with respect to OCP, the concentration of tris(hydroxymethyl)aminomethane in the tris-HCl buffer is preferably 50 mmol/L or more and 300 mmol/L or less, and more preferably 100 mmol/L or more and 200 mmol/L or less. When the concentration of the tris-HCl buffer is less than the lower limit, the pH buffering capacity is lost. If the concentration of the tris-HCl buffer exceeds the upper limit, there is a possibility of cytotoxicity due to the residual of tris(hydroxymethyl)aminomethane.

The pH of the buffer solution supersaturated with respect to OCP is preferably 5.0 or higher and 8.0 or lower, more preferably 6.8 or higher and 8.0 or lower, and even more preferably 7.2 or higher and 7.6 or lower. When the pH is less than the lower limit, OCP dissolves. When the pH exceeds the upper limit, HA precipitates.

### (Second step)

In the case of immersing the base material including OCP in the saturated buffer solution, the mass (mg) of the base material including OCP added per unit volume (mL) of the saturated buffer solution is preferably 0.5 mg or more and 10 mg or less, more preferably 1.0 mg or more and 7.5 mg or less, and even more preferably 3.0 mg or more and 6.0 mg or less, in terms of OCP conversion. When the mass of the base material including OCP is less than the lower limit, the surface area of the base material decreases, and the adsorption amount of the functional molecules decreases. When the mass of the base material including OCP exceeds the upper limit, the adsorption amount per unit area of the base material decreases.

In the case of immersing the base material including OCP in the supersaturated buffer solution, the mass (mg) of the base material including OCP added per unit volume (mL) of the supersaturated buffer solution is preferably 0.5 mg or more and 10 mg or less, more preferably 1.0 mg or more and 7.5 mg or less, and even more preferably 3.0 mg or more and 6.0 mg or less, in terms of OCP conversion. When the mass of the base material including OCP is less than the lower limit, the surface area of the base material decreases, and the adsorption amount of the functional molecules decreases. When the mass of the base material including OCP exceeds the upper limit, the amount of new OCP crystals precipitating on the OCP crystals decreases.

In the case of immersing the base material including OCP in the saturated buffer solution, the immersion time is not particularly limited. For example, the immersion time may be 10 hours or more and 120 hours or less.

In the case of immersing the base material including OCP in the supersaturated buffer solution, the immersion time is not particularly limited. For example, the immersion time may be 10 hours or more and 120 hours or less.

### (Third step)

In the third step, the base material including OCP with the adsorbed functional molecules may be washed by using pure water before the base material including OCP with the adsorbed functional molecules is freeze-dried.

The base material including OCP with the adsorbed functional molecules may be pre-freezed after the base material including OCP with the adsorbed functional molecules is washed and before the base material including OCP with the adsorbed functional molecules is freeze-dried. The temperature for pre-freezing is may be -196°C or higher and -10°C or lower.

The freeze-drying time is not particularly limited, and may be 12 hours or more and 120 hours or less, for example.

According to the bone regeneration material of the embodiment, by using the saturated or supersaturated functional molecule-containing buffer solution for OCP in which the content of functional molecules is adjusted, it is possible to control the adsorption amount of the functional molecules to OCP because the functional molecules are adsorbed to OCP.

### [Bone regeneration material manufacturing device]

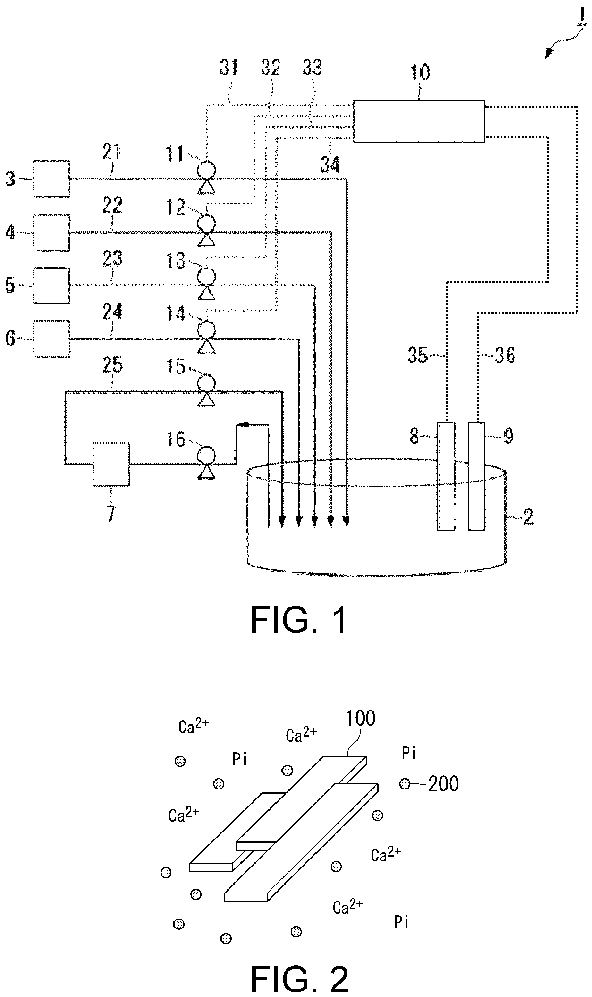

FIG. 1 is a schematic diagram showing a bone regeneration material manufacturing device according to an embodiment of the disclosure.

As shown in FIG. 1, a bone regeneration material manufacturing device 1 of the embodiment includes a reaction vessel 2, an acid/base supply unit 3, a calcium ion supply unit 4, an inorganic phosphate ion supply unit 5, a functional molecule supply unit 6, a functional molecule concentration unit 7, a pH electrode 8, a calcium ion electrode 9, and a control unit 10.

The reaction vessel 2 is a vessel for reacting the base material including OCP with the functional molecules to adsorb the functional molecules to OCP. Specifically, in the reaction vessel 2, a slurry (hereinafter referred to as "slurry") including the base material including OCP with dissolved functional molecules is reacted with a buffer solution that is supersaturated with respect to OCP.

The reaction vessel 2 may include a stirring means for stirring the slurry with the dissolved functional molecules and the buffer solution supersaturated with respect to OCP. Examples of the stirring means include a stirring blade, a magnetic stirrer, etc.

The acid/base supply unit 3 is provided for supplying acid or base to the reaction vessel 2. Acid or base is supplied from the acid/base supply unit 3 to the reaction vessel 2 to adjust the pH of the slurry in the reaction vessel 2. A pump 11 is provided in the pipe 21. The pump 11 is connected to the control unit 10 via a wiring 31.

Examples of the acid include hydrochloric acid, nitric acid, acetic acid, citric acid, etc. Examples of the base include sodium hydroxide, potassium hydroxide, ammonia, etc.

The calcium ion supply unit 4 is provided for supplying calcium ions to the reaction vessel 2. Calcium ions are supplied from the calcium ion supply unit 4 to the reaction vessel 2 to adjust the concentration of the calcium ions in the slurry in the reaction vessel 2. The calcium ion supply unit 4 is connected to the reaction vessel 2 via the pipe 22. A pump 12 is provided in the pipe 22. The pump 12 is connected to the control unit 10 via a wiring 32.

The inorganic phosphate ion supply unit 5 is provided for supplying inorganic phosphate ions to the reaction vessel 2. Inorganic phosphate ions are supplied from the inorganic phosphate ion supply unit 5 to the reaction vessel 2 to adjust the concentration of the inorganic phosphate ions in the slurry in the reaction vessel 2. The inorganic phosphate ion supply unit 5 is connected to the reaction vessel 2 via a pipe 23. A pump 13 is provided in the pipe 23. The pump 13 is connected to the control unit 10 via a wiring 33.

The functional molecules can be provided in the reaction vessel 2 in advance. The functional molecule supply unit 6 is connected to the reaction vessel 2 via a pipe 24. A pump 14 is provided in the pipe 24. The pump 14 is connected to the control unit 10 via a wiring 34. The functional molecules may be supplied from the functional molecule supply unit 6 to the reaction vessel 2 by the pump 14 to dissolve the functional molecules in the slurry in the reaction vessel 2.

The functional molecule concentration unit 7 is provided for recovering and concentrating the functional molecules remaining in the supernatant of the slurry after the functional molecules have been adsorbed onto the base material, and then supplying (reusing) the functional molecules to the reaction vessel 2 again. The functional molecule concentration unit 7 is connected to the reaction vessel 2 via a pipe 25. A pump 15 and a pump 16 are provided in the pipe 25.

The pH electrode 8 is disposed in the reaction vessel 2 and is provided for measuring the pH of the slurry in the reaction vessel 2. The pH electrode 8 is connected to the control unit 10 via a wiring 35.

The calcium ion electrode 9 is disposed in the reaction vessel 2 and is provided for measuring the concentration of the calcium ions in the slurry in the reaction vessel 2. The calcium ion electrode 9 is connected to the control unit 10 via a wiring 36.

In the bone regeneration material manufacturing device 1, the pH of the slurry in the reaction vessel 2 is measured by the pH electrode 8, and the concentration of the calcium ions in the slurry in the reaction vessel 2 is measured by the calcium ion electrode 9. Based on the measurement results (concentrations), the control unit 10 calculates the degree of supersaturation with respect to OCP for the slurry. Based on the calculated degree of supersaturation, the control unit 10 operates the pump 11, the pump 12, and the pump 13 as necessary to supply acid or base, calcium ions, and inorganic phosphate ions to the slurry in the reaction vessel 2 as necessary, so that the degree of supersaturation of the slurry in the reaction vessel 2 becomes a set value.

When the pH and the concentration of calcium ions of the slurry being stirred in the reaction vessel 2 reach predetermined values, the functional molecules are adsorbed onto OCP.

As a result, the bone regeneration material is obtained.

According to the bone regeneration material manufacturing device of the embodiment, based on the pH of the slurry in the reaction vessel 2 measured by the pH electrode 8 and the concentration of the calcium ions in the slurry in the reaction vessel 2 measured by the calcium ion electrode 9, the control unit 10 calculates the degree of supersaturation with respect to OCP. Based on the calculated degree of supersaturation, acid or base, calcium ions, and inorganic phosphate ions are supplied as needed to the slurry in the reaction vessel 2 so that the degree of supersaturation of the slurry in the reaction vessel 2 becomes a set value. Therefore, the adsorption amount of the functional molecules onto OCP can be controlled.

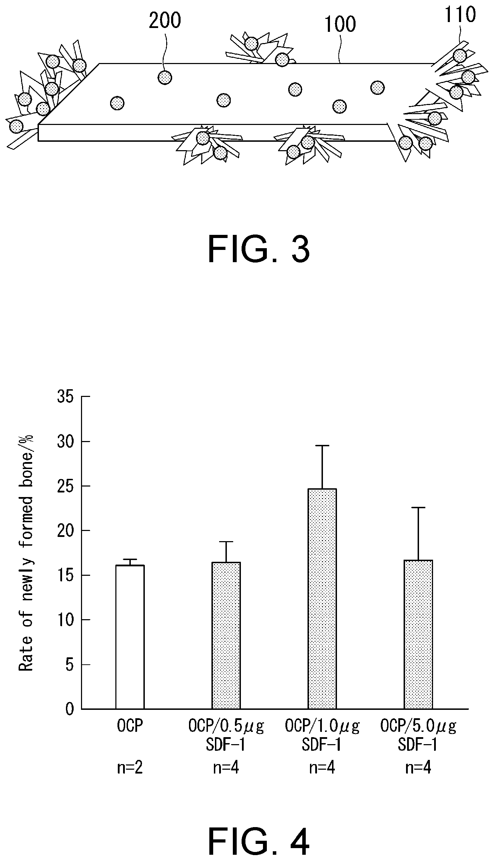

Here, referring to FIG. 2 and FIG. 3, the mechanism for facilitating the adsorption of the functional molecules onto the base material including OCP in the buffer solution supersaturated with respect to OCP in the embodiment is described.

In the buffer solution saturated with respect to OCP, the functional molecules are adsorbed onto the base material including OCP through the interaction between the surface of the base material and the functional molecules.

Meanwhile, as shown in FIG. 2, in the buffer solution supersaturated with respect to OCP, as shown in FIG. 3, new OCP 110 selectively precipitates on the surface of the base material 100, and functional molecules 200 is adsorbed onto the new OCP 110, thereby increasing the adsorption amount of the functional molecules 200.

### [Examples]

The disclosure is further specifically described below with examples. However, the disclosure is not limited to the following examples.

### [Example 1]

Phosphate-buffered saline solutions were prepared by dissolving SDF-1 in 10 mmol/L phosphate-buffered saline. The contents of SDF-1 in the phosphate-buffered saline solutions were set to 0.5 µg per 5 mg of OCP granules (OCP0.5µgSDF-1), 1.0 µg per 5 mg of OCP granules (OCP1.0µgSDF-1), and 5.0 µg per 5 mg of OCP granules (OCP5.0µgSDF-1).

The phosphate-buffered saline solutions were dropped onto 5 mg of OCP granules (particle size ranging from 300 µm to 500 µm) to adsorb SDF-1 onto the OCP granules.

The OCP granules with the adsorbed SDF-1 were pre-frozen at -20°C.

The pre-frozen OCP granules were freeze-dried to obtain a bone regeneration material with SDF-1 adsorbed onto the OCP granules. Three types of bone regeneration materials with different SDF-1 contents were prepared.

A standardized defect with a diameter of 3 mm and a depth of 3 mm was created in the femur of a Sprague-Dawley rat (12 weeks old, male).

5 mg of each of OCP0.5µgSDF-1 (n=4), OCP1.0µgSDF-1 (n=4), and OCP5.0µgSDF-1 (n=4) was implanted into the standardized defect. As a control group, 5 mg of OCP without adsorbed SDF-1 (n=2) was implanted into the standardized defect.

Two weeks after implantation, the femur was extracted, and decalcified tissue sections were prepared.

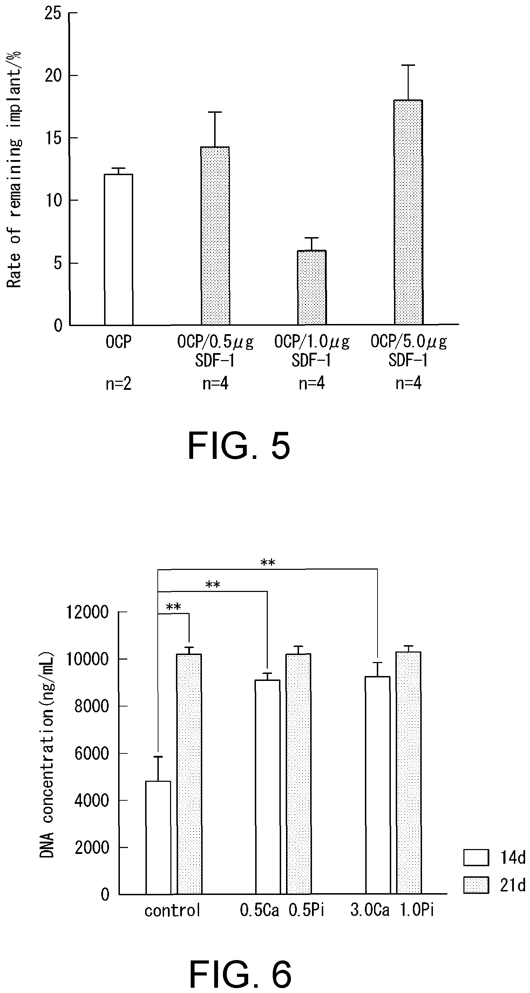

The decalcified tissue sections were stained with hematoxylin-eosin (HE), and the obtained tissue specimens were observed under an optical microscope. From the captured HE tissue images, the newly formed bone area and the remaining OCP granule area were measured, and the ratios of the newly formed bone area and the remaining OCP granule area with respect to the defect area before implantation were calculated, respectively. The results are shown in FIG. 4 and FIG. 5.

In the optical microscope images of the tissue specimens, newly formed bone tissues and remaining OCP granules were observed in the defect area, regardless of whether SDF-1 was adsorbed. FIG. 4 shows the ratio of the newly formed bone area to the defect area. FIG. 5 shows the ratio of the remaining OCP granule area to the defect area.

From the results shown in FIG. 4, the ratio of the newly formed bone area to the defect area was highest for OCP1.0µgSDF-1. Additionally, from the results shown in FIG. 5, the ratio of the remaining OCP granule area to the defect area was lowest for OCP1.0µgSDF-1 and highest for OCP5.0µgSDF-1. The results indicate that for OCP granules with adsorbed SDF-1, there is an optimal adsorption amount (content) of SDF-1 for absorption and replacement with newly formed bone.

### [Example 2]

A buffer solution saturated with respect to OCP (Ca concentration 0.5 mmol/L, inorganic phosphate concentration 0.5 mmol/L, tris-HCl buffer concentration solution 150 mmol/L, pH 7.4, hereinafter referred to as "0.5Ca0.5Pi buffer solution") was prepared, and 200 ng/mL of SDF-1 was dissolved in the buffer solution to prepare an SDF-1-containing buffer solution A. A buffer solution supersaturated with respect to OCP (Ca concentration 3.0 mmol/L, inorganic phosphate concentration 1.0 mmol/L, tris-HCl buffer solution concentration 150 mmol/L, pH 7.4, hereinafter referred to as "3.0Ca1.0Pi buffer solution") was prepared, and 200 ng/mL of SDF-1 was dissolved in the buffer solution to prepare an SDF-1-containing buffer solution B.

Furthermore, as a carrier protein, a bovine serum albumin was dissolved in the SDF-1-containing buffer solution A and the SDF-1-containing buffer solution B at a ratio of 1.0 mg/mL.

5.0 mg of OCP granules (particle size less than 53 µm) were immersed in each of the SDF-1-containing buffer solution A and the SDF-1-containing buffer solution B in which the bovine serum albumin was dissolved to adsorb SDF-1 onto the OCP granules.

The OCP granules with the adsorbed SDF-1 were washed with pure water.

Subsequently, the OCP granules with the adsorbed SDF-1 were freeze-dried for 24 hours to obtain the bone regeneration material with SDF-1 adsorbed onto the OCP granules. As the bone regeneration materials, a bone regeneration material using the 0.5Ca0.5Pi buffer solution (0.5Ca0.5Pi group) and a bone regeneration material using the 3.0Ca1.0Pi buffer solution (3.0Ca1.0Pi group) were prepared.

A D1 cell line is a mouse bone marrow-derived undifferentiated mesenchymal stem cell (MSC). The D1 cell line was seeded at 4×10⁴ cells/well in a 48-well plate together with 5 mg of the 0.5Ca0.5Pi group or the 3.0Ca1.0Pi group, and was cultured in an osteogenic differentiation medium. Additionally, the D1 cell line as a mouse bone marrow-derived undifferentiated mesenchymal stem cell (MSC) was seeded at 4×10⁴ cells/well in a 48-well plate together with 5 mg of OCP granules without adsorbed SDF-1, and cultured in an osteogenic differentiation medium as a control group.

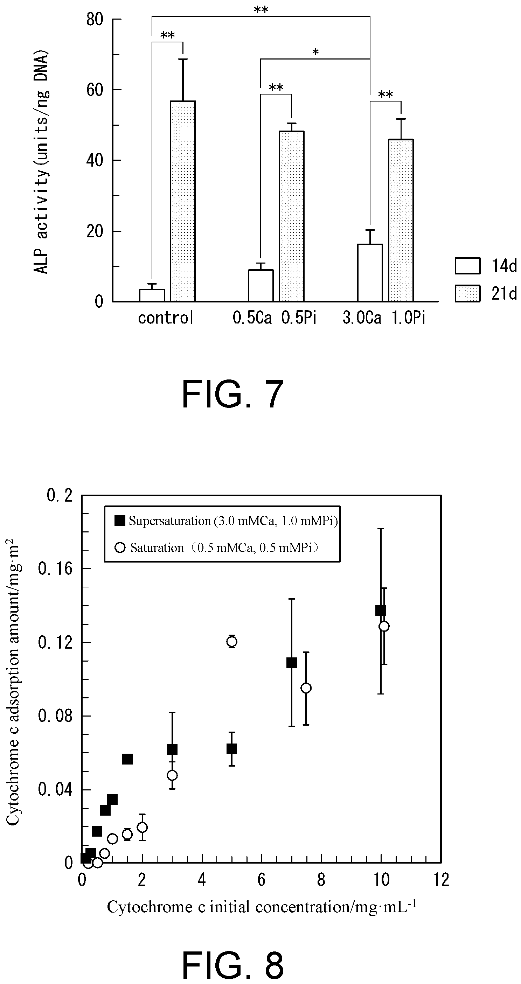

The DNA concentration of the cells and the alkaline phosphatase (ALP) activity were measured on the 14^{th} day the 21^{st} day from the start of culture. ALP is one of the early differentiation markers of osteoblasts. The measured ALP activity was normalized by using the DNA concentration. Data are shown as mean ± standard deviation, and statistical significance was analyzed by conducting a Tukey-Kramer test. A p-value less than 0.05 was considered statistically significant. The results are shown in FIG. 6 and FIG. 7.

From the results shown in FIG. 6, it was found that the DNA concentration on the 14^{th} day from the start of culture showed significantly higher values in the 0.5Ca0.5Pi group and 3.0Ca1.0Pi group than the control group. While the DNA concentrations increased in all groups from the 14^{th} day to the 21^{st} day from the start of culture, no significant difference was observed among the groups.

From the results shown in FIG. 7, it was found that the ALP activity on the 14^{th} day from the start of culture significantly increased in the 3.0Ca1.0Pi group compared to the 0.5Ca0.5Pi group. The 0.5Ca0.5Pi group also showed a tendency for a higher ALP activity than the control group, but no significant difference was observed. The ALP activity increased in all groups from the 14^{th} day to the 21^{st} day from the start of culture, but no significant difference was observed between the groups.

From the results, it was found that the adsorption of SDF-1 onto OCP granules facilitates early proliferation of MSCs on OCP. Furthermore, it was found that the SDF-1 adsorbed onto the OCP granules in the tris-HCl buffer solution supersaturated with respect to OCP facilitates the osteoblastic differentiation of MSCs earlier than the SDF-1 adsorbed onto the OCP granules in the tris-HCl buffer solution saturated with respect to OCP.

### [Example 3]

A bone regeneration material was manufactured by using the bone regeneration material manufacturing device shown in FIG. 1.

The reaction solution was removed from the precipitate of OCP synthesized by a wet process, and the precipitate was washed. Pure water was added to the obtained OCP precipitate to prepare a slurry.

A lysozyme aqueous solution was supplied in advance to the slurry to set the concentration of lysozyme in the slurry to 0.1 mg/mL, and the total amount of slurry in the reaction vessel 2 was set to 1000 mL.

To the slurry including lysozyme, 40 mmol/L of calcium ions were supplied from the calcium ion supply unit 4 by operating the pump 12, and 80 mmol/L of inorganic phosphate ions were supplied from the inorganic phosphate ion supply unit 5 by operating the pump 13. The slurry was stirred at 25°C to adsorb lysozyme onto the OCP. During the process, the measurement of the pH of the slurry in the reaction vessel 2 by the pH electrode 8 and the measurement of the concentration of the calcium ion of the slurry in the reaction vessel 2 by the calcium ion electrode 9 were continued. The total amount of the slurry after the calcium ions and the inorganic phosphate ions were supplied was 2000 mL.

After lysozyme is adsorbed onto the OCP, the OCP and the supernatant were recovered from the slurry.

The adsorption amount of lysozyme to the OCP was estimated by measuring the concentration of lysozyme in the supernatant. Additionally, the crystal structure of the OCP with the adsorbed lysozyme was analyzed by X-ray diffraction.

In the manufacturing of the bone regeneration material, the concentration of the calcium ions in the slurry increased for about 500 seconds after the supply of the calcium ions to the slurry started, and then the concentration of the calcium ions decreased. The concentration of the calcium ions at the time when the supply of the calcium ions to the slurry was stopped was 4.84 mmol/L. Additionally, the concentration of the calcium ions one hour after the supply of the calcium ions to the slurry was stopped was 2.16 mmol/L.

The pH of the slurry was approximately 7 when the supply of the calcium ions to the slurry was initiated. For about 700 seconds after the supply of the calcium ions to the slurry was started, the pH of the slurry decreased to 5.5 and such value was maintained.

24% of the added amount of lysozyme was adsorbed onto the surface of OCP, while 76% remained in the supernatant.

Additionally, it was confirmed that the crystal structure was maintained as OCP after the adsorption of lysozyme.

Based on the above results, it is confirmed that lysozyme can be adsorbed onto OCP while controlling the degree of supersaturation of the slurry by measuring the pH and the concentration of the calcium ions of the slurry by using the bone regeneration material manufacturing device shown in FIG. 1.

### [Example 4]

A cytochrome c-containing buffer solution C was prepared by dissolving cytochrome c at a concentration of 0-10 mg/mL in a buffer solution saturated with respect to OCP (calcium concentration: 0.5 mmol/L, inorganic phosphate concentration: 0.5 mmol/L, tris-HCl buffer concentration: 150 mmol/L, pH 7.4). Additionally, a cytochrome c-containing buffer solution D was prepared by dissolving cytochrome c at a concentration of 0-10 mg/mL in a buffer solution supersaturated with respect to OCP (calcium concentration: 3.0 mmol/L, inorganic phosphate concentration: 1.0 mmol/L, tris-HCl buffer concentration: 150 mmol/L, pH 7.4).

5.0 mg of OCP granules (particle size less than 53 µm) were immersed in each of the cytochrome c-containing buffer solution C and the cytochrome c-containing buffer solution D to adsorb the cytochrome c onto the OCP granules.

After the cytochrome c was adsorbed onto OCP, the OCP and the supernatant were recovered from both the cytochrome c-containing buffer solution C and the cytochrome c-containing buffer solution D.

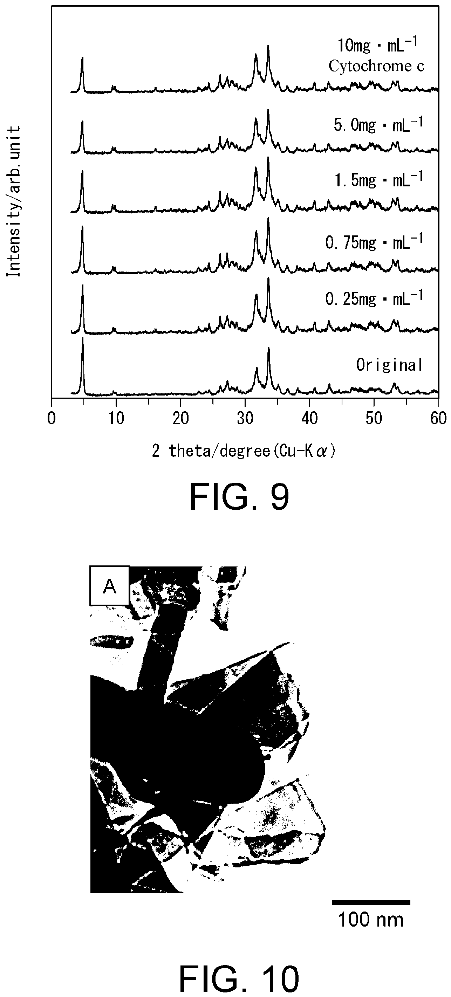

The adsorption amount of the cytochrome c onto OCP was estimated by measuring the concentration of cytochrome c in the supernatant. The results are shown in FIG. 8. Additionally, the crystal structure of the OCP with the adsorbed cytochrome c was analyzed by X-ray diffraction. The results are shown in FIG. 9.

From the results shown in FIG. 8, it was found that in the range of the initial cytochrome c concentration from 0 mg/mL to 3.0 mg/mL, the adsorption amount of cytochrome c was higher in the buffer supersaturated with respect to OCP than in the buffer saturated with respect to OCP. From the results shown in FIG. 9, it was confirmed that the crystal structure of OCP was maintained after the adsorption of the cytochrome c, regardless of the initial concentration of the cytochrome c.

### [Example 5]

A cytochrome c-containing buffer solution E was prepared by dissolving cytochrome c at a concentration of 0.1 mg/mL in a buffer solution supersaturated with respect to OCP (calcium concentration: 2.5 mmol/L, inorganic phosphate concentration: 1.0 mmol/L, tris-HCl buffer concentration: 150 mmol/L, pH 7.4). Additionally, a cytochrome c-containing buffer solution F was prepared by further adding 1.0 mg/mL of the bovine serum albumin to the cytochrome c-containing buffer solution E.

5.0 mg of OCP granules (particle size less than 53 µm) were immersed in each of the cytochrome c-containing buffer solution E and the cytochrome c-containing buffer solution F to adsorb the cytochrome c onto the OCP granules. The OCP granules were immersed in each buffer solution for 3 days. The buffer solution was replaced with a fresh buffer solution on daily basis to adsorb cytochrome c onto the OCP granules.

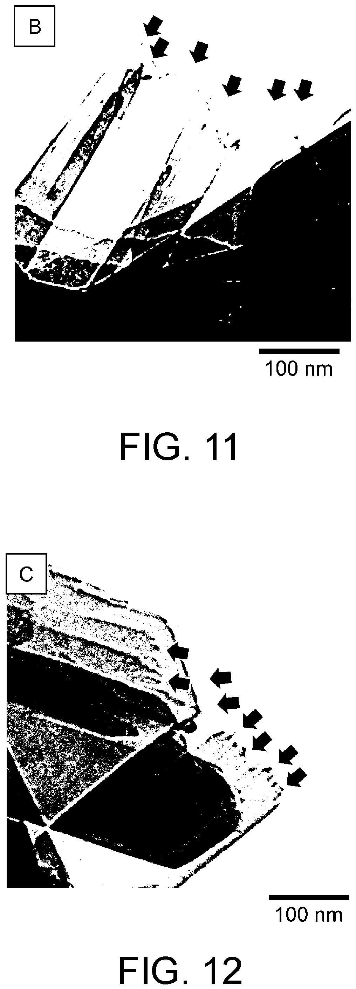

A transmission electron microscope (JEOL JEM-2100F, manufactured by JEOL Ltd.) was used to observe the OCP granules before cytochrome c adsorption, OCP crystals with the cytochrome c adsorbed by using the cytochrome c-containing buffer solution E, and OCP crystals with the cytochrome c adsorbed by using the cytochrome c-containing buffer solution F. The results are shown in FIG. 10 to FIG. 12. FIG. 10 is a transmission electron microscope image of the OCP crystals before cytochrome c adsorption. FIG. 11 is a transmission electron microscope image of the OCP crystals with the cytochrome c adsorbed by using the cytochrome c-containing buffer solution E. FIG. 12 is a transmission electron microscope image of the OCP crystals with the cytochrome c adsorbed by using the cytochrome c-containing buffer solution F.

In FIGs. 11 and 12, as indicated by the arrows, it was found that new OCP crystals were formed at the ends of the OCP crystals.

### [Example 6]

Fetuin was dissolved in a buffer solution saturated with respect to OCP (Ca concentration 0.5 mmol/L, inorganic phosphate ion concentration 0.5 mmol/L, tris-HCl buffer concentration 150 mmol/L, pH 7.4, hereinafter referred to as "0.5Ca0.5Pi buffer") to prepare fetuin-containing 0.5Ca0.5Pi buffer solutions with fetuin concentrations of 0 mg/mL, 0.10 mg/mL, 0.25 mg/mL, 0.50 mg/mL, 0.75 mg/mL, 1.0 mg/mL, or 1.5 mg/mL. Additionally, fetuin was dissolved in a buffer solution supersaturated with respect to OCP (Ca concentration 3.0 mmol/L, inorganic phosphate ion concentration 1.0 mmol/L, tris-HCl buffer concentration 150 mmol/L, pH 7.4, hereinafter referred to as "3.0Ca1.0Pi buffer") to prepare fetuin-containing 3.0Ca1.0Pi buffer solutions with fetuin concentrations of 0 mg/mL, 0.10 mg/mL, 0.25 mg/mL, 0.50 mg/mL, 0.75 mg/mL, 1.0 mg/mL, or 1.5 mg/mL.

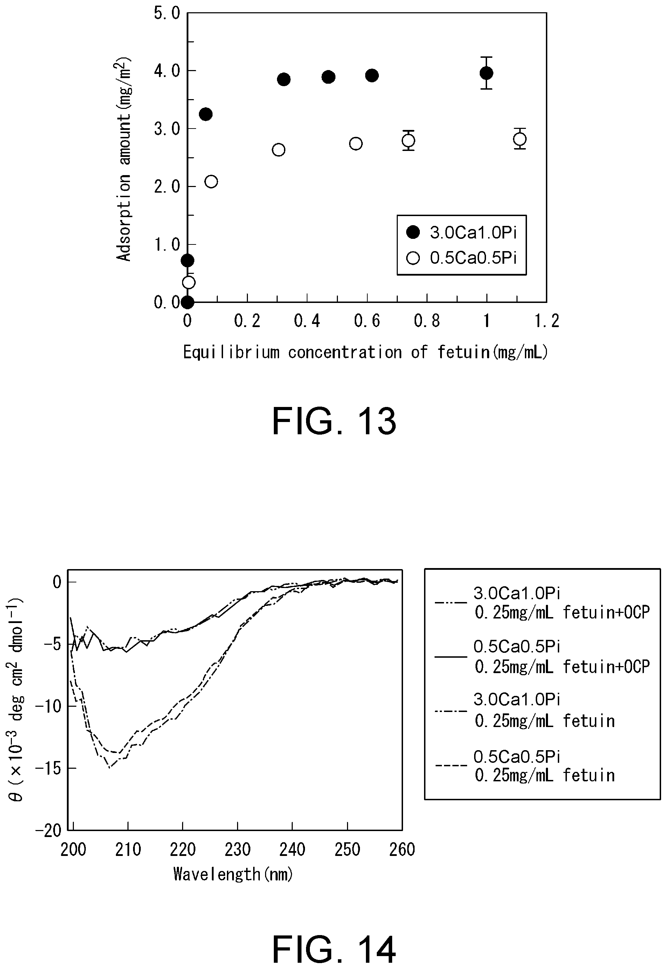

5.0 mg of OCP granules (particle size less than 53 µm) were immersed in each of the fetuin-containing 0.5Ca0.5Pi buffer solutions and fetuin-containing 3.0Ca1.0Pi buffer solutions in which various concentrations of fetuin were dissolved to adsorb fetuin onto the OCP granules. The results are shown in FIG. 13. FIG. 13 is a diagram showing the adsorption isotherms of fetuin onto OCP in the fetuin-containing 0.5Ca0.5Pi buffer solutions and fetuin-containing 3.0Ca1.0Pi buffer solutions.

As shown in FIG. 13, the adsorption amount of fetuin increased in the equilibrium concentration range from 0 to approximately 0.3 mg/mL in the fetuin-containing 0.5Ca0.5Pi buffer solutions as well as the fetuin-containing 3.0Ca1.0Pi buffer solutions.

The adsorption of fetuin tended to saturate in both adsorption isotherms when the equilibrium concentration exceeded 0.3 mg/mL.

The adsorption amounts of fetuin onto OCP immersed in the 3.0Ca1.0Pi buffer solutions were higher than that in the case of immersion in 0.5Ca0.5Pi buffer solutions, regardless of the equilibrium concentration of fetuin.

The adsorption parameters of fetuin to OCP were calculated by approximating to the Langmuir model.

The correlation coefficient was 0.99 or higher for both adsorption isotherms.

The adsorption equilibrium constant for 0.5Ca0.5Pi buffer solutions was 33.5 mL/mg. The adsorption equilibrium constant for 3.0Ca1.0Pi buffer solutions was 69.4 mL/mg.

The saturation adsorption amount for 0.5Ca0.5Pi buffer solutions was 2.9 mg/m². The saturation adsorption amount for 3.0Ca1.0Pi buffer was 4.0 mg/m².

In this manner, both the adsorption equilibrium constant and the saturation adsorption amount were higher for the 3.0Ca1.0Pi buffer solutions than for the 0.5Ca0.5Pi buffer solutions.

The circular dichroism (CD) spectra of fetuin in the 0.5Ca0.5Pi buffer solutions and the 3.0Ca1.0Pi buffer solutions including 0.25 mg/mL fetuin were measured before and after OCP immersion.

A circular dichroism spectropolarimeter (J-805, manufactured by JASCO Corporation) was used for measuring the CD spectra.

FIG. 14 shows the measurement results of the CD spectra.

As shown in FIG. 14, the mean residue ellipticity decreased along with the decrease in fetuin concentration due to adsorption onto OCP.

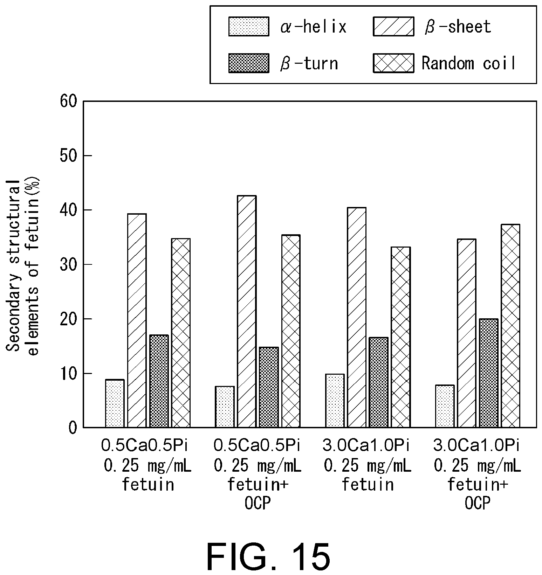

In addition, as shown in FIG. 15, the secondary structure elements of fetuin in 0.5Ca0.5Pi buffer solutions and 3.0Ca1.0Pi buffer solutions were similar before OCP immersion.

As shown in FIGs. 14 and 15, the secondary structure elements of fetuin were maintained after OCP immersion in both 0.5Ca0.5Pi buffer solutions and 3.0Ca1.0Pi buffer solutions.

### [Reference Signs List]

1: Bone regeneration material manufacturing device;
2: Reaction vessel;
3: Acid/base supply unit;
4: Calcium ion supply unit;
5: Inorganic phosphate ion supply unit;
6: Functional molecule supply unit;
7: Functional molecule concentration unit;
8: pH electrode;
9: Calcium ion electrode;
10: Control unit;
11 to 16: Pump;
21 to 25: Pipe;
31 to 36: Wiring.

## Claims

1. A bone regeneration material, formed by a complex, the complex comprising: a base material comprising octacalcium phosphate; and functional molecules, adsorbed to the octacalcium phosphate and being at least one selected from a growth factor and a serum-derived protein.

2. The bone regeneration material as claimed in claim 1, wherein the base material comprises a polylactic-co-glycolic acid copolymer.

3. The bone regeneration material as claimed in claim 1, wherein the growth factor is at least one selected from stromal cell-derived factor 1, bone morphogenetic protein, and vascular endothelial growth factor.

4. The bone regeneration material as claimed in claim 1, wherein the serum-derived protein is at least one selected from fetuin, albumin, and fibronectin.

5. A method for manufacturing a bone regeneration material, comprising:
preparing a phosphate-buffered saline solution by dissolving functional molecules in a phosphate-buffered saline, the functional molecules being at least one selected from a growth factor and a serum-derived protein;
bringing the phosphate-buffered saline solution into contact with a base material comprising octacalcium phosphate to adsorb the functional molecules to the octacalcium phosphate; and
freeze-drying the base material comprising the octacalcium phosphate with the adsorbed functional molecules.

6. The method for manufacturing the bone regeneration material as claimed in claim 5, wherein the growth factor is at least one selected from stromal cell-derived factor 1, bone morphogenetic protein, and vascular endothelial growth factor.

7. The method for manufacturing the bone regeneration material as claimed in claim 5, wherein the serum-derived protein is at least one selected from fetuin, albumin, and fibronectin.

8. A method for manufacturing a bone regeneration material, comprising:
preparing a functional molecule-containing buffer solution by dissolving functional molecules in a buffer solution that is saturated or supersaturated with respect to octacalcium phosphate, the functional molecules being at least one selected from a growth factor and a serum-derived protein;
immersing a base material comprising octacalcium phosphate in the functional molecule-containing buffer solution to adsorb the functional molecules to the octacalcium phosphate; and
freeze-drying the base material comprising the octacalcium phosphate with the adsorbed functional molecules.

9. The method for manufacturing the bone regeneration material as claimed in claim 8, wherein the growth factor is at least one selected from stromal cell-derived factor 1, bone morphogenetic protein, and vascular endothelial growth factor.

10. The method for manufacturing the bone regeneration material as claimed in claim 8, wherein the serum-derived protein is at least one selected from fetuin, albumin, and fibronectin.

11. A bone regeneration material manufacturing device (1), comprising:
a reaction vessel (2), accommodating a slurry comprising a base material that comprises octacalcium phosphate;
an acid/base supply unit (3), supplying acid or base to the slurry in the reaction vessel (2);
a calcium ion supply unit (4), supplying calcium ions to the slurry in the reaction vessel (2);
an inorganic phosphate ion supply unit (5), supplying inorganic phosphate ions to the slurry in the reaction vessel (2);
a functional molecule supply unit (6), supplying functional molecules to the slurry in the reaction vessel (2), the functional molecules being at least one selected from a growth factor and a serum-derived protein;
a functional molecule concentration unit (7), after the functional molecules are adsorbed onto the base material in the reaction vessel (2), recovering and concentrating remaining functional molecules in a supernatant of the slurry among the functional molecules, and supplying the recovered and concentrated functional molecules to the reaction vessel (2) again;
a pH electrode (8), measuring a pH of the slurry in the reaction vessel (2);
a calcium ion electrode (9), measuring a concentration of calcium ions of the slurry in the reaction vessel (2); and
a control unit (10), calculating a degree of supersaturation with respect to the octacalcium phosphate for the slurry based on the pH of the slurry in the reaction vessel (2) measured by the pH electrode (8) and the concentration of the calcium ions of the slurry in the reaction vessel (2) measured by the calcium ion electrode (9), and, based on the degree of supersaturation, transmitting an instruction to the acid/base supply unit (3), the calcium ion supply unit (4), and the inorganic phosphate ion supply unit (5) to supply the acid or base, the calcium ions, and the inorganic phosphate ions to the slurry in the reaction vessel (2), so that the degree of supersaturation of the slurry in the reaction vessel (2) becomes a set value.

12. The bone regeneration material manufacturing device (1) as claimed in claim 11, wherein the growth factor is at least one selected from stromal cell-derived factor 1, bone morphogenetic protein, and vascular endothelial growth factor.

13. The bone regeneration material manufacturing device (1) as claimed in claim 11, wherein the serum-derived protein is at least one selected from fetuin, albumin, and fibronectin.
